Europäisches Patentamt

European Patent Office

Office européen des brevets

(11) Veröffentlichungsnummer: **0 054 180**
**A2**

(12) **EUROPÄISCHE PATENTANMELDUNG**

(21) Anmeldenummer: 81109488.7

(22) Anmeldetag: 03.11.81

(51) Int. Cl.³: **C 07 D 487/04**
C 07 C 101/18, C 07 C 79/12
C 07 C 79/22, C 07 C 79/36
C 07 C 47/55, C 07 C 63/04
//(C07D487/04, 239/00, 235/00)

(30) Priorität: 16.12.80 CH 9267/80
07.09.81 CH 5753/81

(43) Veröffentlichungstag der Anmeldung:
23.06.82 Patentblatt 82/25

(84) Benannte Vertragsstaaten:
AT BE CH DE FR GB IT LI NL

(71) Anmelder: F. HOFFMANN-LA ROCHE & CO.
Aktiengesellschaft

CH-4002 Basel(CH)

(72) Erfinder: Chodnekar, Madhukar Subraya, Dr.
Rebhaldenstrasse 6
CH-4411 Seltisberg(CH)

(72) Erfinder: Kienzle, Frank, Dr.
Tannwaldweg 9
CH-4113 Flüh(CH)

(74) Vertreter: Mahé, Jean et al,
Postfach 3255 Grenzacherstrasse 124
CH-4002 Basel(CH)

(54) Neues Verfahren zur Herstellung eines Imidazochinazolinonderivates und neue Zwischenprodukte.

(57) Neues Verfahren zur Herstellung des 7-Brom-1,5-dihydro-3,6-dimethylimidazo[2,1-b]chinazolin-2(3H)-ons in D-Form oder als Racemat durch Umsetzung des N-(6-Amino-3-brom-2-methylbenzyl)-alanin-äthylesters in D-Form oder als Racemat mit Bromcyan.

EP 0 054 180 A2

Croydon Printing Company Ltd.

F.Hoffmann-La Roche & Co. Aktiengesellschaft,Basel,Schweiz

0054180

RAN 4044/57

## Neues Verfahren zur Herstellung eines Imidazochinazolinonderivates und neue Zwischenprodukte

Die vorliegende Erfindung betrifft ein neues Verfahren zur Herstellung eines Imidazochinazolinonderivatives, nämlich des 7-Brom-1,5-dihydro-3,6-dimethylimidazo[2,1-b]chinazolin-2(3H)-ons in D-Form oder als Racemat. Diese Verbindung zeichnet sich, wie in der europäischen Patentanmeldung Nr. 21,338 ausgeführt wird, durch eine die Blutplättchenaggregation hemmende Aktivität aus und kann demgemäss zur Verhütung von Thrombosen Verwendung finden.

Das erfindungsgemässe Verfahren ist dadurch gekennzeichnet, dass man N-(6-Amino-3-brom-2-methylbenzyl)-alanin-äthylester in D-Form oder als Racemat mit Bromcyan umsetzt.

Diese Umsetzung wird zweckmässig in einem Lösungsmittel, wie einem niederen Alkanol, z.B. Aethanol, durchgeführt. Zur Herstellung des D-7-Brom-1,5-dihydro-3,6-dimethylimidazo[2,1-b]chinazolin-2(3H)-on geht man von N-(6-Amino-3-brom-2-methylbenzyl)-D-alanin-äthylester aus und führt die Umsetzung zweckmässig unter Abkühlen durch.

Mé/22.9.81

Die Herstellung des racemischen Verfahrensproduktes kann auch bei höheren Temperaturen, z.B. Rückflusstemperaturen des Reaktionsgemisches, erfolgen.

Der im obigen Verfahren verwendete Ausgangsester wird vorzugsweise dadurch hergestellt, dass man den 3-Brom-2-methyl-6-nitrobenzylalkohol chloriert, das so erhaltene 3-Brom-2-methyl-6-nitrobenzylchlorid in den N-(3-Brom-2-methyl-6-nitrobenzyl)-alanin-äthylester in D-Form oder als Racemat überführt und diesen reduziert.

Die oben genannten Zwischenprodukte N-(6-Amino-3-brom-2-methylbenzyl)-alanin-äthylester und N-(3-Brom-2-methyl-6-nitrobenzyl)-alanin-äthylester in D-Form oder als Racemat, 3-Brom-2-methyl-6-nitrobenzylchlorid, 3-Brom-2-methyl-6-nitrobenzylalkohol, sowie die weiter unten erwähnten Substanzen, 3-Brom-2-methylbenzaldehyd, 3-Brom-2-methylbenzoesäure und 3-Brom-6-nitro-o-tolualdehyd sind neu und als solche ebenfalls Gegenstand der vorliegenden Erfindung.

Die Chlorierung des 3-Brom-2-methyl-6-nitrobenzyl-alkohols wird zweckmässig mit Thionylchlorid, Phosphoroxychlorid oder Phosphortrichlorid, in einem Lösungsmittel, wie Dimethylformamid oder einem tertiären Amin, z.B. Triäthylamin oder Pyridin, und gewünschtenfalls einem Aether, z.B. Diäthyläther oder Tetrahydrofuran, bei einer Temperatur zwischen etwa 0 und 50°C, vorzugsweise unter Abkühlung, durchgeführt.

Die Ueberführung des 3-Brom-2-methyl-6-nitrobenzyl-chlorids in den N-(3-Brom-2-methyl-6-nitrobenzyl)-alanin-äthylester in D-Form oder als Racemat wird zweckmässig mit Alanin-äthylester in D-Form oder als Racemat, in einem Lösungsmittel, z.B. einem niederen Alkanol, wie Aethanol, einem Aether, wie Tetrahydrofuran, oder Dimethylformamid, bei einer Temperatur zwischen Raumtemperatur und Rückflusstemperatur, jedoch unter 100°C, bewerkstelligt.

Die Reduktion des N-(3-Brom-2-methyl-6-nitrobenzyl)-alanin-äthylesters in D-Form oder als Racemat wird zweckmässig durch Hydrierung in Gegenwart eines Katalysators, wie Raney-Nickel, in einem Lösungsmittel, wie Aethanol, durchgeführt.

Der weiter oben verwendete 3-Brom-2-methyl-6-nitro-benzylalkohol wird zweckmässig ausgehend von 3-Brom-2-methylbenzaldehyd über 3-Brom-6-nitro-o-tolualdehyd hergestellt.

Das 3-Brom-2-methylbenzaldehyd kann entweder ausgehend von 3-Brom-2-methylanilin oder ausgehend von 2-Methyl-3-nitrobenzylalkohol über 3-Amino-2-methylbenzylalkohol und 3-Brom-2-methylbenzylalkohol hergestellt werden.

Das letztgenannte Zwischenprodukt, der 3-Brom-2-methylbenzylalkohol, kann ausgehend von der käuflichen 3-Amino-2-methylbenzoesäure über die 3-Brom-2-methylbenzoesäure hergestellt werden.

Beispiel 1

Eine auf 0°C abgekühlte Lösung von 4 g N-(6-Amino-3-brom-2-methylbenzyl)-D-alanin-äthylester in 30 ml Aethanol wird mit einer Lösung von 1,44 g Bromcyan in 5 ml Aethanol versetzt. Das Reaktionsgemisch wird bei Raumtemperatur gerührt, dann gekühlt und mit konz. Ammoniak alkalisch (pH 9-10) gestellt. Nach 2 Stunden Rühren wird das Gemisch abgenutscht. Das erhaltene D-7-Brom-1,5-dihydro-3,6-dimethylimidazo[2,1-b]chinazolin-2(3H)-on wird nacheinander mit Aethanol, Wasser, Aceton und Diäthyläther gewaschen und im Vakuum bei 70°C getrocknet. Smp. > 300°C (Zersetzung), $[\alpha]_D$: -34° (c = 1% Trifluoressigsäure), Ausbeute: 60% d. Th.

A)    Das N-(6-Amino-3-brom-2-methylbenzyl)-D-alanin-äthylester kann wie folgt hergestellt werden:

a)    Einer Lösung von 28,1 g 3-Brom-2-methyl-6-nitrobenzylalkohol in 300 ml Diäthyläther werden unter Kühlen und Rühren 55 ml Dimethylformamid gefolgt von 30 ml Thionylchlorid zugetropft. Das Gemisch wird bei 5°C gerührt und dann auf Eis/Wasser gegossen. Die wässerige Lösung wird abgetrennt und mit Diäthyläther extrahiert. Die vereinigten Aetherlösungen werden mit ges. NaCl-Lösung gewaschen, getrocknet und eingedampft. Der Rückstand wird im Vakuum getrocknet. Man erhält 3-Brom-2-methyl-6-nitrobenzylchlorid, Smp. 76-77°C nach Umkristallisation aus Aethylacetat/Hexan; Ausbeute: 95% d. Th.

b)    Zu einer Lösung vom 2,53 g D-Alanin-äthylester-hydrochlorid in 15 ml Aethanol, wird bei Raumtemperatur eine Lösung von 10 ml Triäthylamin in 15 ml Aethanol getropft. Das Reaktionsgemisch wird auf 60°C erwärmt und mit einer Lösung von 4 g 3-Brom-2-methyl-6-nitrobenzylchlorid in 15 ml Aethanol versetzt. Das Reaktionsgemisch wird bei 80°C gerührt und dann zur Trockene eingedampft. Der Rückstand wird mit 50 ml Wasser versetzt und mit Methylenchlorid extrahiert. Der Extrakt wird mit Wasser gewaschen, getrocknet und eingedampft. Eine Lösung des erhaltenen N-(3-Brom-2-methyl-6-nitrobenzyl)-D-alanin-äthylesters in 60 ml Methylenchlorid wird mit Kohle behandelt, filtriert und eingedampft; $[\alpha]_D$: -29,5° (c = 1% Chloroform); Ausbeute: 90% d. Th.

c)   Eine Lösung von 4,7 g N-(3-Brom-2-methyl-6-nitrobenzyl)-D-alanin-äthylester in 25 ml Aethanol und 5 ml Triäthylamin wird in Gegenwart von Raney-Nickel hydriert. Dann wird das Gemisch vom Katalysator abfiltriert und das Filtrat zur Trockene eingedampft. Man erhält N-(6-Amino-3-brom-2-methylbenzyl)-D-alanin-äthylester, $[\alpha]_D$: + 10,2° (c = 1% in Aethanol), Ausbeute: 93% d. Th.

B)   Das 3-Brom-2-methyl-6-nitrobenzylalkohol kann wie folgt hergestellt werden:

a)   Einem Gemisch von 274 ml 98% $HNO_3$ und 234 ml konz. Schwefelsäure werden unter Rühren bei 5°C 66 g 3-Brom-2-methylbenzaldehyd zugetropft. Nach Rühren bei 5°C wird das Gemisch auf 2,5 l Eis/Wasser gegossen. Das ausfallende Gemisch von 3-Brom-6-nitro- und 3-Brom-5-nitro-o-tolualdehyd wird abfiltriert, in Aethylacetat gelöst und getrocknet. Die Lösung wird auf 500 ml eingeengt. Nach Zugabe von 500 ml Hexan und Kristallisation werden 28,8 g reines 3-Brom-6-nitro-o-tolualdehyd, Smp. 94-96°C, abfiltriert. Aus der Mutterlauge werden noch 5,5 g reines Produkt kristallisiert. Totalausbeute: 34,5 g (42%).

b)   Eine Suspension von 28,3 g 3-Brom-6-nitro-o-tolualdehyd in 500 ml Aethanol wird unter Rühren und Kühlen mit 2,1 g Natriumborhydrid versetzt. Dann wird die Lösung auf 200 ml eingeengt und auf Eis/Wasser gegossen. Das Produkt wird abfiltriert und getrocknet. Ausbeute: 28,1 g (98%) 3-Brom-2-methyl-6-nitrobenzylalkohol, Smp. 67-68°C nach Umkristallisation aus Aethylacetat/Hexan.

C)   Das 3-Brom-2-methylbenzaldehyd kann wie folgt ausgehend von 3-Brom-2-methylanilin hergestellt werden:


Einer Lösung von 92 g 3-Brom-2-methylanilin in 100 ml
Wasser und 114 ml konz. Salzsäure werden 200 g Eis zugegeben. Dem auf -5°C abgekühlten Gemisch wird unter Rühren
eine Lösung von 35 g $NaNO_2$ in 50 ml Wasser unter die Oberfläche getropft, wobei man eine Diazoniumsalzlösung erhält.
23 g Paraformaldehyd und 52,6 g Hydroxylaminhydrochlorid
in 340 ml Wasser werden nach Zusatz von 102 g Natriumacetat erhitzt bis eine klare Lösung entsteht. Zu dieser
Lösung werden 13 g $CuSO_4$ x $5H_2O$, 2 g $Na_2SO_3$ und eine Lösung
von 320 g Natriumacetat (x$3H_2O$) in 360 ml Wasser gegeben.
Zur erhaltenen Lösung wird unter Rühren bei 15-20°C die
Diazoniumsalzlösung unter die Oberfläche eingetropft. Man
rührt bei Raumtemperatur und nach Zugabe von 460 ml konz.
Salzsäure bei 110°C. Nach Wasserdampfdestillation erhält
man das 3-Brom-2-methylbenzaldehyd. Das Destillat wird
neutralisiert und mit Diäthyläther extrahiert. Die Aetherlösung wird getrocknet, filtriert und eingedampft. Zum
Rückstand wird 40%ige Natriummetabisulfitlösung gegeben.
Die nach Schütteln entstandene Suspension wird auf 0°C
abgekühlt und abgenutscht. Der Rückstand wird mit Wasser
und dann Diäthyläther gewaschen und dann in Wasser suspendiert. Zu dieser Suspension wird konz. Salzsäure gegeben.
Das Gemisch wird erhitzt, dann abgekühlt und mit Diäthyläther extrahiert. Die Aetherlösung wird mit ges. Kochsalzlösung gewaschen, getrocknet und eingedampft. Der Rückstand
wird getrocknet. Man erhält 66 g (67%) reines 3-Brom-2-
methylbenzaldehyd in Form eines Oels.


D)   Das 3-Brom-2-methylbenzaldehyd kann auch wie folgt
ausgehend von 2-Methyl-3-nitrobenzylalkohol hergestellt
werden:


a)   Eine Lösung von 50,1 g 2-Methyl-3-nitrobenzylalkohol
in 180 ml Aethanol wird in Gegenwart von 2,5 g 10%igem
Pd/C hydriert. Dann wird das Gemisch vom Katalysator abfiltriert. Das Filtrat wird zur Trockene eingedampft. Der Rückstand wird aus Aethanol kristallisiert, genutscht, mit Diäthyläther gewaschen und getrocknet. Man erhält 3-Amino-2-methylbenzylalkohol, Smp. 113-115°C. Ausbeute: 93% d. Th.

b) Einem auf 0°C abgekühlten Gemisch von 13,7 g 3-Amino-2-methylbenzylalkohol, 200 ml Wasser und 20 ml 63%iger rauchender Bromwasserstoffsäure wird eine Lösung von 7 g Natriumnitrit in 50 ml Wasser zugetropft. Nach Rühren wird bei 0°C portionenweise eine Suspension von 28,6 Kupfer (I) bromid in 125 ml Wasser zugegeben und das Reaktionsgemisch nacheinander bei 10°C, bei Raumtemperatur und bei 100°C gerührt. Nach dem Abkühlen wird das Reaktionsgemisch mit Methylenchlorid extrahiert. Der Extrakt wird mit Wasser gewaschen, getrocknet und eingedampft. Der Rückstand wird aus Diäthyläther/Hexan kristallisiert. Man erhält 3-Brom-2-methylbenzylalkohol, Smp. 103-106°C, Ausbeute: 60% d. Th.

c) Ein Gemisch von 13,8 g 3-Brom-2-methylbenzylalkohol, 200 ml Methylenchlorid und 70 g Mangandioxyd wird gerührt, dann vom Mangandioxyd abgenutscht. Das Filtrat wird zur Trockene eingedampft und der Rückstand wird über Kieselgel mit Methylenchlorid als Laufmittel gereinigt. Man erhält 3-Brom-2-methylbenzaldehyd als farbloses Oel, Ausbeute: 70% d. Th.

E) Das 3-Brom-2-methylbenzaldehyd kann auch wie folgt ausgehend von 3-Amino-2-methylbenzoesäure hergestellt werden:

a) Einer Lösung von 133 g 3-Amino-2-methylbenzoesäure in 1,8 l Wasser und 120 ml 63% HBr wird unter Kühlen eine Lösung von 62 g Natriumnitrit in 500 ml Wasser zugetropft. Die erhaltene Diazoniumlösung wird bei 0-5°C gerührt und dann unter die Oberfläche einer Lösung von 140 g Kupfer(I)-bromid in 400 ml Wasser und 200 ml 63% HBr unter Rühren langsam eingepumpt. Der dabei auftretende Schaum wird durch Zugabe von Aether kontrolliert. Dann wird bei Raum-

temperatur gerührt, auf 20 l Wasser gegossen und mit Methylenchlorid extrahiert. Die organische Phase wird filtriert, mit Wasser gewaschen, getrocknet und eingedampft. Der Rückstand wird in 200 ml Essigester suspendiert, mit 200 ml Hexan versetzt und abfiltriert. Ausbeute: 134 g 3-Brom-2-methylbenzoesäure (71%), Smp. 151-153°C.

b) Eine Lösung von 134 g 3-Brom-2-methylbenzoesäure in 800 ml trockenem Aethylenglykoldimethyläther (Monoglyme) wird portionenweise mit 24 g gepulvertem Natriumborhydrid versetzt. Dann werden unter Kühlen 104 ml Bortrifluorid-ätherat zugetropft und das Gemisch wird bei Raumtemperatur gerührt. Danach werden 100 ml Wasser zugetropft und dann wird das Gemisch auf eine Lösung von 100 g Natriumbicarbonat in 10 l Wasser gegossen, dann das Gemisch mit Methylenchlorid extrahiert, die organische Phase mit Wasser gewaschen, getrocknet und eingedampft. Der Rückstand wird aus Essigester/Hexan umkristallisiert. Ausbeute: 99,5 g 3-Brom-2-methylbenzylalkohol (80%), Smp. 103-105°C.

c) Zu einer Lösung von 99 g 3-Brom-2-methylbenzylalkohol in 1,5 l Methylenchlorid werden unter Rühren 500 g aktives Mangandioxid gegeben. Nach Rühren bei Raumtemperatur wird das Gemisch vom Mangandioxid abfiltriert, mit Methylenchlorid gewaschen und das Filtrat wird eingedampft. Ausbeute: 93 g 3-Brom-2-methylbenzaldehyd (95%) als farbloses Oel.

## Beispiel 2

Anstatt wie im Beispiel 1 den Ausgangsester in D-Form mit Bromcyan bei 0°C umzusetzen, wird die Bromierung des racemischen Esters, N-(6-Amino-3-brom-2-methylbenzyl)-D,L-alanin-äthylester, bei Rückflusstemperatur durchgeführt. Unter den sonst gleichen Bedingungen wie in Beispiel 1 und in etwa gleicher Ausbeute erhält man das D,L-7-Brom-1,5-dihydro-3,6-dimethylimidazo[2,1-b]chinazolin-2(3H)-on, Smp. > 300°C (Zersetzung).

Der Ausgangsester, N-(6-Amino-3-brom-2-methylbenzyl)-D,L-alanin-äthylester, wird dadurch hergestellt, dass man in Analogie zu Beispiel 1A) b) und 1A) c) D,L-Alanin-äthylester-hydrochlorid mit 3-Brom-2-methyl-6-nitrobenzyl-chlorid zu N-(3-Brom-2-methyl-6-nitrobenzyl)-D,L-alanin-äthylester, das in 90% d. Th. Ausbeute als Oel erhalten wird, umsetzt und diesen Ester in Gegenwart von Raney-Nickel in 93% d. Th. Ausbeute hydriert.

Patentansprüche

1. Verfahren zur Herstellung des 7-Brom-1,5-dihydro-3,6-dimethylimidazo[2,1-b]chinazolin-2(3H)-on in D-Form oder als Racemat, dadurch gekennzeichnet, dass man N-(6-Amino-3-brom-2-methylbenzyl)-alanin-äthylester in D-Form oder als Racemat mit Bromcyan umsetzt.

2. Verfahren nach Anspruch 1, dadurch gekennzeichnet, dass man den Ausgangsester durch Chorierung des 3-Brom-2-methyl-6-nitrobenzylalkohols, Ueberführung des so erhaltenen 3-Brom-2-methyl-6-nitrobenzylchlorids in N-(3-Brom-2-methyl-6-nitrobenzyl)-alanin-äthylester in D-Form oder als Racemat und Reduktion davon herstellt.

3. N-(6-Amino-3-brom-2-methylbenzyl)-alanin-äthylester in D-Form oder als Racemat.

4. N-(3-Brom-2-methyl-6-nitrobenzyl)-alanin-äthylester in D-Form oder als Racemat.

5. 3-Brom-2-methyl-6-nitrobenzylchlorid.

6. 3-Brom-2-methyl-6-nitrobenzylalkohol.

7. 3-Brom-6-nitro-o-tolualdehyd.

8. 3-Brom-2-methylbenzaldehyd.

9. 3-Brom-2-methylbenzoesäure.

***